# EUROPÄISCHE PATENTANMELDUNG

(11) **EP 0 703 223 A2**
(43) Veröffentlichungstag der Anmeldung: **27.03.1996**
(21) Anmeldenummer: 95113646.4
(22) Anmeldetag: 31.08.1995
(51) Int. Cl.: C07D 239/70, A61K 7/13

(54) **Haarfärbemittel**

(30) Priorität: 24.09.1994 DE 4434165
(71) Anmelder: HOECHST AKTIENGESELLSCHAFT, D-65926 Frankfurt am Main (DE)
(72) Erfinder: Akram, Mustafa Dr., D-22457 Hamburg (DE)

(57) **Zusammenfassung**

Die vorliegende Erfindung betrifft die Verwendung einer Verbindung der allgemeinen Formel I 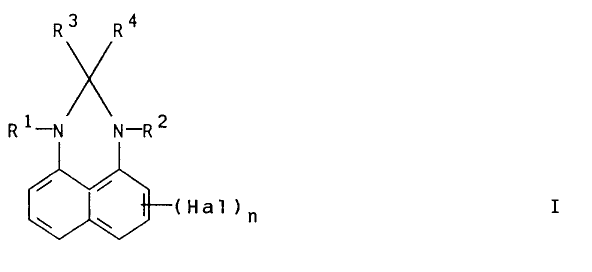
worin R¹ bis R⁴, Hal und n wie in Anspruch 1 angegeben definiert sind, als Kupplersubstanz bei der Herstellung von Oxidationsfarbstoffen sowie Haarfärbemittel, die eine Verbindung der allgemeinen Formel I enthalten.

## Beschreibung

Die vorliegende Erfindung betrifft die Verwendung von 2,3-Dihydroperimidin-Derivaten als Kupplersubstanz bei der Herstellung von Oxidationsfarbstoffen sowie Haarfärbemittel, die ein 2,3-Dihydroperimidin-Derivat enthalten.

Beim oxidativen Färben von Keratinfasern werden in der Faser durch Reaktion von Kuppler- mit Entwicklersubstanzen in Gegenwart von Oxidationsmitteln Farbstoffe bzw. Pigmente erzeugt.

An solche Oxidationsfarbstoffe werden zahlreiche anwendungstechnische Anforderungen gestellt, insbesondere im Hinblick auf Lichtechtheit, Säureechtheit, Schweißechtheit, Reibechtheit, Dauerwellenechtheit, Waschechtheit, Thermostabilität, Aufzieh- und Ausgleichsvermögen sowie Farbstärke. Außerdem sollen sie toxikologisch und dermatologisch unbedenklich sein. Insbesondere aufgrund ihrer hohen Farbstärke und guten Echtheitseigenschaften haben sie eine besondere Bedeutung erlangt (siehe z.B. J.F. Corbett, in K. Venkataraman, The Chemistry of Synthetic Dyes, Vol. V, 478-505 (1971); J.F. Corbett, Rev. Progr. Coloration, Vol.V, 52-58 (1985)).

In der Praxis werden Kuppler- und Entwicklersubstanzen in Mischung mit geeigneten Hilfsstoffen als Haarfärbemittel angeboten, aus denen dann der Farbstoff in der Keratinfaser gebildet wird.

Zur Erzeugung von blauen Farbtönen werden vorwiegend 1,3-Diaminobenzol-Derivate als Kuppler und 1,4-Diaminobenzol-Derivate als Entwicklersubstanzen eingesetzt.

Schwarze und schwarzbraune Farbtöne werden aus den zunächst erhaltenen Farbstoffen durch Mischungen mit anderen Kupplerkomponenten, beispielsweise mit 3-Aminophenol- und/oder 1,3-Dihydroxybenzol-Derivaten, erhalten.

Überraschenderweise wurde nun gefunden, daß man neben blonden Farbtönen auch schwarze bis schwarzbraune Färbungen auf direktem Weg erhalten kann, wenn man 2,3-Dihydroperimidin-Derivate als Kupplersubstanzen einsetzt.

Die vorliegende Erfindung betrifft demnach die Verwendung einer Verbindung der allgemeinen Formel I 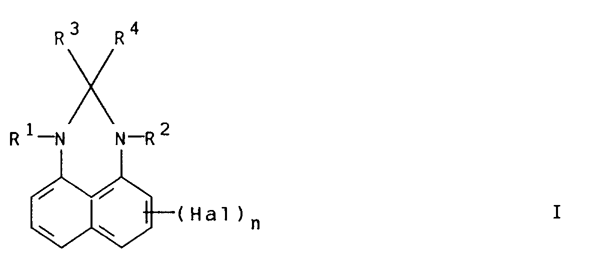
worin
R¹ und R² unabhängig voneinander Wasserstoff, (C₁-C₄)-Alkyl, Hydroxy-(C₁-C₃)-alkyl, 2,3-Dihydroxypropyl, 2-Hydroxy-3-(C₁-C₄)-alkoxypropyl, Phenyl oder substituiertes Phenyl,
R³ und R⁴ unabhängig voneinander Wasserstoff, Trichlormethyl, (C₁-C₆)-Alkyl, Di-(C₁-C₂)-alkoxymethyl, Hydroxy-(C₁-C₄)-alkyl, Phenyl oder substituiertes Phenyl,
Hal Fluor, Chlor oder Brom und
n 0, 1 oder 2
bedeuten,
als Kupplersubstanz bei der Herstellung von Oxidationsfarbstoffen.

Alkylgruppen können geradkettig oder verzweigt sein und bedeuten beispielsweise Methyl, Ethyl, i-Propyl, n-Propyl, n-Butyl, i-Butyl, sec-Butyl, tert-Butyl, Pentyl oder Hexyl. Analoges gilt für Alkoxygruppen.
Substituiertes Phenyl kann 1, 2 oder 3 gleiche oder verschiedene Substituenten tragen. Geeignete Substituenten sind Hydroxy, (C₁-C₄)-Alkyl, (C₁-C₄)-Alkoxy, Fluor, Chlor, Brom oder auch der Methylendioxyrest, der gleichzeitig zwei Wasserstoffatome des Phenylrests substituiert.
R¹ und R² stehen bevorzugt unabhängig voneinander für Wasserstoff, Methyl, 1-Hydroxyethyl, 2-Hydroxyethyl, 2-Hydroxypropyl, 3-Hydroxypropyl oder Phenyl,
R³ und R⁴ stehen bevorzugt unabhängig voneinander für Wasserstoff, Trichlormethyl, (C₁-C₆)-Alkyl, Hydroxymethyl oder substituiertes Phenyl.
Hal steht bevorzugt in 4- und/oder 6-Stellung des Perimidingerüstes und bedeutet bevorzugt Chlor.

Die Verbindungen der allgemeinen Formel I werden bevorzugt in Mischung mit einer oder mehreren Entwicklersubstanzen in sogenannte Haarfärbemittel eingearbeitet.

Die vorliegende Erfindung betrifft demnach auch ein Haarfärbemittel, enthaltend eine Kupplersubstanz und eine Entwicklersubstanz, dadurch gekennzeichnet, daß es als Kupplersubstanz eine Verbindung der allgemeinen Formel I enthält.

Die erfindungsgemäßen Haarfärbemittel enthalten die Verbindungen der allgemeinen Formel I in neutraler Form oder in Salzform in einer für eine Färbung ausreichenden Menge, vorzugsweise 0,01 bis 5 Gew.-%, besonders bevorzugt 0,1 bis 3 Gew.-% bezogen auf das Gesamtgewicht des Mittels. Dabei können die Verbindungen der allgemeinen Formel I allein oder in Mischung untereinander eingesetzt werden.

Die erfindungsgemäßen Haarfärbemittel können neben den Verbindungen der allgemeinen Formel I auch weitere Kupplersubstanzen oder Mischungen weiterer Kupplersubstanzen enthalten, wobei in der Regel Mengen von 0,01 bis 5 Gew.-%, vorzugsweise 0,1 bis 3 Gew.-% bezogen auf das Gesamtgewicht des Mittels eingesetzt werden.

Geeignete weitere Kupplersubstanzen sind beispielsweise 1,3-Dihydroxybenzol, 2-Methyl-1,3-dihydroxybenzol, 2-Amino-4-(2'-hydroxyethylamino)-anisol, 2-Amino-4-(2'-hydroxyethylamino)-phenetol, 2-Amino-4-ethylaminoanisol, 2,4-Diaminobenzylalkohol, 2,4-Diamino-phenoxyethanol, 1,3-Diaminobenzol, 3-Aminophenol, 3-Amino-2-methylphenol, 4-Amino-2-hydroxyphenoxyethanol, 4-Hydroxy-1,2-methylendioxybenzol, 4-(2'-Hydroxyethylamino)-1,2-methylendioxybenzol, 2,4-Diamino-5-ethoxytoluol, 4-Hydroxyindol, 5,6-Dihydroxyindol, 1-Naphthol, 1,5-Dihydroxynaphthalin.

Die in den erfindungsgemäßen Haarfärbemitteln enthaltenen Entwicklersubstanzen liegen bevorzugt in Mengen von 0,01 bis 5 Gew.-%, besonders bevorzugt 0,1 bis 3 Gew.-% bezogen auf das Gesamtgewicht des Mittels vor. Auch die Entwicklersubstanzen können alleine oder in Mischung untereinander eingesetzt werden.

Geeignete Entwicklersubstanzen sind beispielsweise 1,4-Diaminobenzol, 2,5-Diaminotoluol, 2,5-Diaminobenzylalkohol, 4-Aminophenol, 4-Amino-3-methylphenol, 2,5-Diaminopyridin, Tetraaminopyrimidin und deren physiologisch verträglichen Salze.

Die erfindungsgemäßen Haarfärbemittel können gegebenenfalls noch andere Farbkomponenten enthalten und zwar insbesondere direktziehende Haarfarbstoffe und/oder Anthrachinonfarbstoffe und/oder Azofarbstoffe. Geeignete Farbkomponenten der genannten Stoffklassen sind beispielsweise bei K. Venkataraman, The Chemistry of Synthetic Dyes, Vol.V, Seiten 507 - 529 (1971) beschrieben. Die genannten anderen Farbkomponenten sind in den erfindungsgemäßen Haarfärbemitteln beispielsweise in Mengen von 0,01 bis 5 Gew.% bezogen auf das Gesamtgewicht des Mittels enthalten.

Die erfindungsgemäßen Haarfärbemitteln liegen vorteilhafterweise in Form von kosmetischen Zubereitungen, beispielsweise als Cremes, Emulsionen oder Gele, vor, welche neben den genannten Oxidationsfarbstoff-Vorprodukten in der Kosmetik übliche Hilfsstoffe enthalten. Übliche Hilfsmittel sind beispielsweise anionische oder nicht-ionogene Emulgiermittel, Verdickungsmittel, sowie Antioxidantien wie Ascorbinsäure, Thioglykolsäure oder Natriumsulfit.

Je nach Zusammensetzung können die erfindungsgemäßen Haarfärbemittel schwach sauer, neutral oder alkalisch reagieren. Bevorzugt weisen sie einen pH-Wert von 8,0 bis 11,5 auf, wobei die pH-Einstellung beispielsweise mit Ammoniak, Kaliumcarbonat, Natriumhydroxid oder Kaliumhydroxid erfolgen kann.

Die erfindungsgemäßen Haarfärbemittel können in einfacher Weise durch Mischen einer oder mehrerer Verbindungen der allgemeinen Formel I mit einer oder mehreren Entwicklersubstanzen und gegebenenfalls weiterer Kupplersubstanzen, weiterer Farbkomponenten und/oder geeigneter Hilfsstoffe und gegebenenfalls Einstellen des gewünschten pH-Werts hergestellt werden.

Die oxidative Färbung, d.h. die Reaktion der genannten Kuppler- und Entwicklersubstanzen kann grundsätzlich mit Luftsauerstoff erfolgen, gegebenenfalls unter Zusatz an sich bekannter Katalysatoren. Bevorzugt werden aber als Oxidationsmittel Wasserstoffperoxid, beispielsweise als 6%ige wäßrige Lösung, dessen Anlagerungsprodukte an Harnstoff oder Melamin, Natriumperborat, Kaliumperoxidisulfat oder Gemische der genannten Verbindungen eingesetzt.

In der Praxis wird beispielsweise ein erfindungsgemäßes Haarfärbemittel kurz vor dem Gebrauch mit einem der genannten Oxidationsmittel gemischt und auf das Haar aufgetragen. Die Anwendungstemperaturen bewegen sich dabei im Bereich von 15 bis 40^{o}C. Nach einer Einwirkungsdauer von ca. 30 Minuten wird das restliche Mittel vom Haar durch Spülen entfernt und das Haar mit einem milden Shampoo nachgewaschen und getrocknet.

Die Verbindungen der allgemeinen Formel I sind zum Teil bekannt und beispielsweise in Compt. Rend. 252, 899 (1961), Chem. Heterocycl. Compd. 14, 1145 (1978), Chem. Heterocycl. Compd. 14, 1156 (1978), J. Heterocycl. Chem. 5, 591 (1968), Pharm. Chem. J. 12, 899 (1978), Compt. Rend. 224, 1569 (1947) oder Chem. Heterocycl. Compd. 10, 484 (1974) beschrieben, zum Teil aber auch noch neu.

Die vorliegende Erfindung betrifft auch Verbindungen der allgemeinen Formel I worin
R¹ und R² beide Wasserstoff, beide Hydroxy-(C₁-C₃)-alkyl oder R¹ (C₁-C₄)-Alkyl und R² Hydroxy-(C₁-C₃)-alkyl,
R³ und R⁴ beide Wasserstoff, beide Hydroxy-(C₁-C₃)-alkyl oder R³ (C₁-C₄)-Alkyl und R⁴ Hydroxy-(C₁-C₃)-alkyl,
Hal Fluor, Chlor oder Brom und
n 0,1 oder 2
bedeuten, wobei R¹, R², R³ und R⁴ nicht gleichzeitig für Wasserstoff stehen können, wenn n = 0.

Die Verbindungen der allgemeinen Formel I können beispielsweise durch Umsetzung von 1,8-Diaminonaphthalin-Derivaten der allgemeinen Formel II 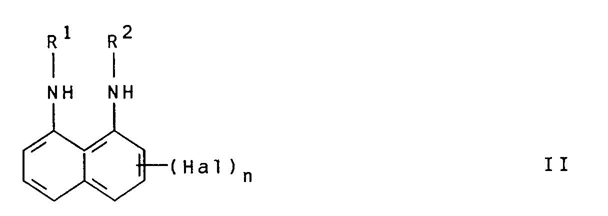
mit Carbonylverbindungen der allgemeinen Formel III 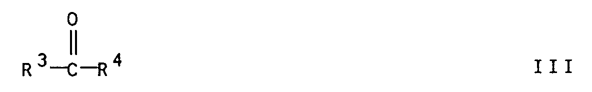
wobei R¹ bis R⁴, Hal und n wie oben angegeben definiert sind, hergestellt werden (siehe beispielsweise Compt. Rend. 252, 899 (1961), Chem. Heterocycl. Compd. 14, 1156 (1978), J. Heterocycl. Chem. 5, 591 (1968), Pharm. Chem. J. 12, 899 (1978), Compt. Rend. 224, 1569 (1947)). Geeignete Verbindungen der allgemeinen Formel II sind beispielsweise 1,8-Diaminonaphthalin, 1-Methylamino-8-aminonaphthalin, 1-Phenylamino-8-amino-naphthalin 2-Chlor-1,8-diaminonaphthalin und 1,8-Bis-(2-hydroxyethylamino)-naphthalin.

Geeignete Verbindungen der allgemeinen Formel III sind beispielsweise Formaldehyd, Acetaldehyd, Propionaldehyd, Butyraldehyd, Isobutyraldehyd, Aceton, Methylethylketon, Diethylketon, Methyl-propylketon, Ethyl-isopropylketon, Methyl-isobutylketon, 3-Methoxybutyraldehyd, Hydroxyaceton, Dihydroxyaceton, Diacetonalkohol, 1,5-Dihydroxy-2-methylpentanon-3, Glycerinaldehyd, Glyoxal-monodimethylacetal, Glyoxal-monodiethylacetal, Benzaldehyd, Piperonal, Vanillin und Salicylaldehyd.

Verbindungen der allgemeinen Formel I mit n = 1 oder 2 können darüberhinaus durch Umsetzung von Verbindungen der allgemeinen Formel I mit n = 0 mit beispielsweise Sulfurylchlorid erhalten werden (Chem. Heterocycl. Compd. 14, 1145 (1978)).

Verbindungen der allgemeinen Formel I können darüberhinaus durch Reduktion entsprechender Perimidin-Derivate, 1,3-Dialkylperimidinium-Salze oder 1,3-Dialkylperimidin-2-one mit Lithiumaluminiumhydrid oder Natriumborhydrid erhalten werden (Chem. Heterocycl. Compd. 10, 485 (1974)).

Die Verbindungen der allgemeinen Formel I können gegebenenfalls in Form von Säureadditionsverbindungen vorliegen. Besonders bevorzugt sind Hydrochloride und Hydrosulfate.

Die aus den Verbindungen der allgemeinen Formel I und an sich bekannten Entwicklersubstanzen erhaltenen Oxidationsfarbstoffe weisen hervorragende anwendungstechnische Eigenschaften auf, insbesondere im Hinblick auf Ziehvermögen, Farbstärke und Deckkraft, sowie Waschechtheit, Säureechtheit und Thermostabilität.

Die Verbindungen der allgemeinen Formel I weisen darüberhinaus als Bestandteil der erfindungsgemäßen Haarfärbemittel eine ausgezeichnete Lagerstabilität auf.

Die nachfolgenden Beispiele dienen zur Erläuterung des Erfindungsgegenstandes, ohne ihn auf die genannten Beispiele zu beschränken.

### Beispiel 1: 2-Methyl-2-hydroxymethyl-2,3-dihydroperimidin, hydrosulfat

Man legt eine Mischung von 79,1 g 1,8-Diaminonaphthalin, 57,4 g Schwefelsäure 96%ig und 700 ml Wasser bei 50^{o}C vor und dosiert in 10 Minuten 57,8 g Hydroxyaceton zu. Anschließend rührt man 2 Stunden bei 50^{o}C nach, kühlt auf 0^{o}C, isoliert das Produkt durch Filtration und trocknet bei 70^{o}C im Vakuum.
Ausbeute: 71,6 g graues Kristallpulver
Schmelzpunkt: 115 - 118^{o}C.

In analoger Weise können hergestellt werden:

### Beispiel 2: 1,3-Bis-(2-hydroxyethyl)-2,3-dihydroperimidin, hydrosulfat

Schmelzpunkt: >200^{o}C

### Beispiel 3: 2,2-Bis-hydroxymethyl-2,3-dihydroperimidin, hydrosulfat

Schmelzpunkt: 150 - 153^{o}C

### Beispiel 4: Haarfärbemittel in Cremeform

1,98 g 2,2-Dimethyl-2,3-dihydroperimidin
1,85 g p-Phenylendiamin·2HCl
1,20 g Ölsäure
0,50 g Natriumdithionit
6,20 g Laurylalkoholdiglykolethersulfat, Natriumsalz (28%ige Lsg.)
18,0 g Cetyl-Stearylalkohol
7,50 g Ammoniak, 25%
Wasser auf 100
60 g des vorstehend genannten Haarfärbemittels werden kurz vor dem Gebrauch mit 60 g Hydrogenperoxidlösung 6%ig gemischt. Man läßt das Gemisch 35 Min bei 40^{o}C auf hellbraune Naturhaare mit 40% Grauanteil einwirken. Danach wird die Farbmasse ausgespült, nachshampooniert und das Haar getrocknet.
Das Haar hat einen gleichmäßigen Mittelbraunton mit einem Violett-Charakter erhalten.

### Beispiel 5: Haarfärbemittel in Gelform

1,98 g 2,2-Dimethyl-2,3-dihydroperimidin
0,10 g p-Aminophenol
12,0 g Ölsäure
12,0 g Isopropanol
5,00 g Nonoxynol-4
10,0 g Ammoniak, 25 %
0,5 g Natriumsulfit, wasserfrei
Wasser auf 100
50 g des vorstehend genannten Färbemittels werden kurz vor dem Gebrauch mit 75 g Hydrogenperoxidlösung 6 %ig gemischt. Man läßt das Gemisch 30 Min bei 35^{o}C auf mittelblonde Naturhaare einwirken. Danach wird die Farbmasse ausgespült, nachshampooniert und das Haar getrocknet.
Das Haar ist in einem intensiven Dunkelgoldblond eingefärbt.

### Beispiel 6 Haarfärbemittel in Cremeform

3,20 g 2-Methyl-2-hydroxymethyl-2,3-dihydroperimidin, hydrosulfat
1,98 g p-Phenylendiamin·2 HCl
0,12 g m-Aminophenol
2,00 g Ölsäure
0,10 g Polyacrylsäure
0,50 g Natriumsulfit, wasserfrei
4,00 g Laurylalkoholdiglykolethersulfat, Natriumsalz (28%ige Lsg.)
8,00 g Ammoniak, 25 %
Wasser auf 100
50 g des vorstehend genannten Haarfärbemittels werden kurz vor dem Gebrauch mit 50 g Hydrogenperoxidlösung 6%ig gemischt. Man läßt das Gemisch 30 Min bei 25^{o}C auf hellblonde Naturhaare mit 50% Grauanteil einwirken. Danach wird die Farbmasse ausgespült, nachshampooniert und das Haar getrocknet.
Es hat einen intensiven Graphitschwarzton erhalten.

### Beispiel 7: Haarfärbemittel in Gelform

3,20 g 2-Methyl-2-hydroxymethyl-2,3-dihydroperimidin, hydrosulfat
2,2 g 2,5-Diaminotoluolsulfat
0,35 g 3-Nitro-4-aminophenol
14,0 g Ölsäure
10,0 g Isopropanol
2,00 g PEG-3-Cocamine
10,0 g Ammoniak, 25 %
0,50 g Ascorbinsäure
Wasser auf 100
40 g des vorstehend genannten Färbemittels werden kurz vor dem Gebrauch mit 60 g Hydrogenperoxidlösung 6%ig gemischt. Man läßt das Gemisch 30 min bei 40^{o}C auf hellblonde Naturhaare einwirken. Danach wird die Farbmasse ausgespült, nachshampooniert und das Haar getrocknet. Das Haar ist in einem intensiven Graphitton mit leicht rötlichem Reflex eingefärbt.

### Beispiel 8: Haarfärbemittel in Cremeform

3,38 g 2,2-Bis-hydroxymethyl-2,3-dihydroperimidin, hydrosulfat
1,20 g p-Aminophenol
0,30 g m-Aminophenol
0,25 g HC Red No. 3
0,05 g m-Phenylendiamin
2,50 g Laurylethersulfat Natriumsalz (70 %ige Paste)
1,00 g Ölsäure
0,60 g Natriumsulfit, wasserfrei
12,0 g Cetylalkohol
6,00 g Myristylalkohol
1,00 g Propylenglykol
10,0 g Ammoniak, 25 %
Wasser auf 100
60 g des vorstehend genannten Färbemittels werden kurz vor dem Gebrauch mit 60 g Hydrogenperoxidlösung 6%ig gemischt. Man läßt das Gemisch 30 Min bei 40^{o}C auf hellblonde Naturhaare mit 20 % Grauanteil einwirken. Danach wird die Farbmasse ausgespült, nachshampooniert und das Haar getrocknet.
Das Haar ist in einem Mokkabraun eingefärbt.

### Beispiel 9: Haarfärbemittel in Gelform

3,38 g 2,2-Bis-hydroxymethyl-2,3-dihydroperimidin, hydrosulfat
1,98 g p-Phenylendiamin·2HCl
6,00 g Nonoxynol-4
14,0 g Ölsäure
1,50 g PEG-3-Cocamine
14,0 g Isopropanol
10,0 g Ammoniak, 25 %
0,45 g Natriumsulfit, wasserfrei
Wasser auf 100
40 g des vorstehend genannten Färbemittels werden kurz vor dem Gebrauch mit 60 g Hydrogenperoxidlösung 6%ig gemischt. Man läßt das Gemisch auf 100% ergrautes Naturhaar 30 Min bei 40^{o}C einwirken. Danach wird das Färbemittel ausgespült, nachshampooniert und das Haar getrocknet.
Das Haar hat einen intensiven Dunkelrauchgrauton erhalten.

Analog den Beschreibungen der Beispiel 4 bis 9 können erfindungsgemäße Haarfärbemittel auch mit folgenden Verbindungen hergestellt werden:

| Beispiel | - 2,3 - Dihydroperimidin | Schmelzpunkt(^{o}C) |
|---|---|---|
| 10 | 2-Methyl | 70 - 72 |
| 11 | 2-Trichlormethyl | 85 - 87 |
| 12 | 2-Ethyl | 77 - 79 |
| 13 | 2-Diethoxymethyl | 65 - 66 |
| 14 | 2-Propyl | 78 - 80 |
| 15 | 2-Hexyl | 57 - 58 |
| 16 | 1-Phenyl | 97 - 99 |
| 17 | 1-Phenyl-2-methyl | > 200 |
| 18 | 2-(3',4'-Methylendioxyphenyl) | 155 - 157 |
| 19 | 2-(2'-Hydroxy-3'-methoxyphenyl) | 187 - 189 |
| 20 | 2-(2'-Hydroxyphenyl) | 196 - 198 |
| 21 | 4-Chlor-2,2-dimethyl | 108 - 110 |
| 22 | 1-Methyl ,hydrosulfat | >200 |
| 23 | 1,3-Dimethyl | 145 - 149 |
| 24 | 1-(2,3-Dihydroperimidin-1-yl)-ethanol | 80 - 83 |
| 25 | 1(2,3-Dihydroperimidin-1-yl)-propanol-2 | 74 - 75 |
| 26 | 4,6-Dichlor-1,3-dimethyl- | 122 - 124 |
| 27 | 2,2-Dimethyl | 115 - 117 |
| 28 | 2,2-H | 75 - 77 |

## Patentansprüche

1. Verwendung einer Verbindung der allgemeinen Formel I 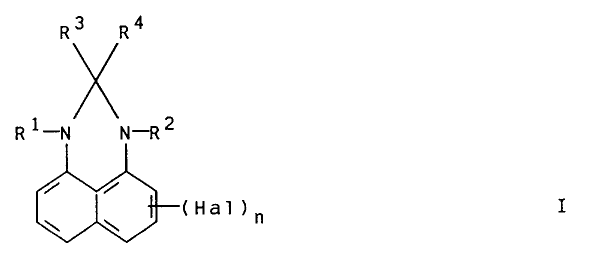 worin
R¹ und R² unabhängig voneinander Wasserstoff, (C₁-C₄)-Alkyl, Hydroxy-(C₁-C₃)-alkyl, 2,3-Dihydroxypropyl, 2-Hydroxy-3-(C₁-C₄)-alkoxypropyl, Phenyl oder substituiertes Phenyl,
R³ und R⁴ unabhängig voneinander Wasserstoff, Trichlormethyl, (C₁-C₆)-Alkyl, Di-(C₁-C₂)-alkoxymethyl, Hydroxy-(C₁-C₄)-alkyl, Phenyl oder substituiertes Phenyl,
Hal Fluor, Chlor oder Brom und
n 0, 1 oder 2
bedeuten,
als Kupplersubstanz bei der Herstellung von Oxidationsfarbstoffen

2. Verwendung gemäß Anspruch 1, dadurch gekennzeichnet, daß R¹ und R² unabhängig voneinander für Wasserstoff, Methyl, 1-Hydroxyethyl, 2-Hydroxyethyl, 2-Hydroxypropyl, 3-Hydroxypropyl oder Phenyl stehen.

3. Verwendung gemäß Anspruch 1 und/oder Anspruch 2, dadurch gekennzeichnet, daß R³ und R⁴ unabhängig voneinander für Wasserstoff, Trichlormethyl, (C₁-C₆)-Alkyl, Hydroxymethyl oder substituiertes Phenyl stehen.

4. Verwendung gemäß einem oder mehreren der Ansprüche 1 bis 3, dadurch gekennzeichnet, daß Hal in 4- und/oder 6-Stellung des Perimidingerüstes steht und Chlor bedeutet.

5. Haarfärbemittel, enthaltend eine Kupplersubstanz und eine Entwicklersubstanz, dadurch gekennzeichnet, daß es als Kupplersubstanz eine Verbindung der allgemeinen Formel I gemäß einem oder mehreren der Ansprüche 1 bis 4 enthält.

6. Haarfärbemittel gemäß Anspruch 5, dadurch gekennzeichnet, daß es die Verbindung der allgemeinen Formel I in Mengen von 0,01 bis 5 Gew.-%, besonders bevorzugt 0,1 bis 3 Gew.-% bezogen auf das Gesamtgewicht des Mittels enthält.

7. Haarfärbemittel gemäß Anspruch 5 und/oder 6, dadurch gekennzeichnet, daß es die Entwicklersubstanz in Mengen von 0,01 bis 5 Gew.-%, besonders bevorzugt 0,1 bis 3 Gew.-%, bezogen auf das Gesamtgewicht des Mittels enthält.

8. Haarfärbemittel gemäß einem oder mehreren der Ansprüche 5 bis 7, dadurch gekennzeichnet, daß es weitere Farbkomponenten enthält.

9. Haarfärbemittel gemäß einem oder mehreren der Ansprüche 5 bis 8, dadurch gekennzeichnet, daß es in Form einer kosmetischen Zubereitung vorliegt.

10. Verbindungen der allgemeinen Formel I des Anspruchs 1, worin R¹ und R² beide Wasserstoff, beide Hydroxy-(C₁-C₃)-alkyl oder R¹ (C₁-C₄)-Alkyl und R² Hydroxy-(C₁-C₃)-alkyl, R³ und R⁴ beide Wasserstoff, beide Hydroxy-(C₁-C₃)-alkyl oder R³ (C₁-C₄)-Alkyl und
R⁴ Hydroxy-(C₁-C₃)-alkyl, Hal, Fluor, Chlor oder Brom und
n 0,1 oder 2
bedeuten, wobei R¹, R², R³ und R⁴ nicht gleichzeitig für Wasserstoff stehen können, wenn n = 0.
